# NEW EUROPEAN PATENT SPECIFICATION

(11) **EP 2 173 356 B2**
(45) Date of publication and mention of the opposition decision: **02.05.2018**
(45) Mention of the grant of the patent: 16.04.2014
(21) Application number: 08785441.0
(22) Date of filing: 07.08.2008
(51) Int. Cl.: A61K 31/593, A61P 21/00

(54) **USE OF 25-HYDROXY VITAMIN D3 TO INCREASE MUSCLE MASS IN MAMMALS**
VERWENDUNG VON 25-HYDROXY-VITAMIN D3 ZUR ERHÖHUNG DER MUSKELMASSE BEI SÄUGERN
UTILISATION DE LA 25-HYDROXYVITAMINE D3 POUR AUGMENTER LA MASSE MUSCULAIRE CHEZ LES MAMMIFÈRES

(30) Priority: 07.08.2007 EP 07015458
(43) Date of publication of application: 14.04.2010
(73) Proprietor: DSM IP Assets B.V., 6411 TE Heerlen (NL)
(72) Inventor: CHUNG, Thau, Kiong, Singapore 588134 (SG); OLIVEROS, Benito, Averion, Laguna 4031 (PH); PENALBA, Francisco, Feranil, Laguna 4031 (PH)
(74) Representative: Schwander, Kuno
(86) International application number: PCT/EP2008/006537
(87) International publication number: WO 2009/019027

(56) References cited:
- WO-A1-2007/059960
- WO-A2-2007/047327
- VISSER MARJOLEIN; DEEG DORLY J H; LIPS PAUL: "Low vitamin D and high parathyroid hormone levels as determinants of loss of muscle strength and muscle mass (sarcopenia): The Longitudinal Aging Study Amsterdam" JOURNAL OF CLINICAL ENDOCRINOLOGY & METABOLISM, vol. 88, no. 12, December 2003 (2003-12), pages 5766-5772, XP002502110
- AGUADO P; GONZALEZ-CASAUS M L; COBO T; DEL CAMPO T; MARTINEZ M E; MARTIN-MOLA E: "The efficacy of two dosing regimens (daily colecalciferol versus cyclical calcidiol) for the prevention of bone mass loss in postmenopausal women with Vitamin D insufficiency" JOURNAL OF BONE AND MINERAL RESEARCH, vol. 21, no. Suppl.1, September 2006 (2006-09), page S306, XP002502111
- BIRGE S J; HADDAD J G: "25-HYDROXY CHOLECALCIFEROL STIMULATION OF MUSCLE METABOLISM" JOURNAL OF CLINICAL INVESTIGATION, vol. 56, no. 5, 1975, pages 1100-1107, XP002502112
- SJÖSTRÖM M; LORENTZON R; LARSSON S E; HOLMLUND D: MEDICAL BIOLOGY, vol. 56, no. 4, 1978, pages 209-215, XP009107816 Helsinki

## Description

### FIELD OF THE INVENTION

This invention relates to a method of increasing muscle mass in mammals by administration of 25-Hydroxy Vitamin D3.

### BACKGROUND OF THE INVENTION

While the many benefits of supplementing a diet with Vitamin D are known, especially regarding bone health, little is known about Vitamin D and its derivatives' effect on other bodily systems.

In many mammals, muscle mass is an important characteristic, both in terms of general health and well being of the animal, as well as for its economic and/or agricultural characteristics. It would thus be beneficial to provide a mechanism to increase not only bone strength, but also muscle mass.

### DESCRIPTION OF THIS INVENTION

It has been found, in accordance with this invention, that myogenesis can be enhanced by prenatally administering to a mammal a sufficient quantity of 25-hydroxy vitamin D3.

As used throughout the specification and claims, "25-OH D3" means 25-hydroxy vitamin D3.

In mammals, the number of muscle fibers is determined prenatally and remains constant throughout postnatal life. There are two types of muscle fibers: primary and secondary. During muscle fiber hyperplasia (an increase in the number of cells or fibers), primary myoblasts develop first, then primary myoblasts fuse to form primary fibers. Next comes a longer period of secondary myoblast development (secondary fiber hyperplasia) on the surface of these primary fibers. As would be expected, while all mammals follow this general plan of development, each species has its own timeline.

For example, in pigs, primary fiber hyperplasia begins at approximately day 35 of gestation and continues to day 60. Secondary fiber hyperplasia starts at about day 50 of gestation and lasts until about day 90. In essence, skeletal muscle fiber formation is essentially complete after day 90 of gestation. Similarly, primary muscle fiber appears at gestation day 32 in sheep, day 60 in bovines and day 56 in humans. Secondary muscle fiber begins to form at gestation day 38, 90 and 90 of gestation in sheep, bovines and humans, respectively.

Postnatal skeletal muscle growth in the pig occurs through muscle fiber hypertrophy (increase in the size of the cell) and is associated with an increase in the DNA content. The terminally differentiated myofibers are incapable of mitosis and therefore the myonuclei do not retain the ability to synthesize DNA. Satellite cells, which are myogenic precursor cells present in the skeletal muscle cells, are the postnatal source of DNA that contribute to growing muscle fibers. DNA accretion occurs through proliferation of satellite cells followed by differentiation and fusion with existing muscle fibers, resulting in dramatic increases in muscle cell size and nuclear content.

The increase in satellite cell-derived nuclei is proportional to and a prerequisite for muscle protein accretion, i.e. muscle growth. Satellite cells become committed to their particular developmental program during late embryonic development. Located beneath the basal lamina, satellite cells are capable of proliferating, differentiation and joining existing fibers for fusing with each other to form new fibers. At birth, skeletal muscle is made up of about 10% proliferating satellite cells; however, they decrease rapidly toward the end of the growth period.

The development of skeletal muscle in mammals begins during early embryonic stages and continues throughout their lives. Skeletal myogenesis is initiated in the embryo as a result of signaling molecules from surrounding tissues that specify myogenic cell fate. The muscle regulatory factor (MRF) gene family, including Myf5, MyoD, Myogenin and MRF4 which are specifically expressed in skeletal muscle cells, play important roles in regulating skeletal muscle development and growth. MyoD and Myf5 determine the precursor cells to myogenic cell fates and the formation of myoblasts during embryogenesis. Myogenin and MRF4 involves in the terminal differentiation of myoblasts into myotubes.

Satellite cells are first apparent towards the end of embryogenesis and serve as a primary source for the myogenic cells required for postnatal muscle growth, repair and maintenance of skeletal muscle. They are normally quiescent located between the sarcolemma and basement membrane of terminally-differentiated mature skeletal muscle fibers. In response to stimulus such as postnatal muscle growth (muscle hypertrophy) and myotrauma, satellite cells become activated, proliferate and differentiate. Both muscle regulatory factors and growth factors (GFs) are implicated in the activation, proliferation and differentiation of satellite cells. Growth factors include fibroblast growth factor (FGF), insulin-like growth factors, members of the transforming growth factor (TGF) superfamily (myostatin and TGF-□1) and hepatocyte growth factor (HGF).

While not wishing to be bound by theory, it appears that dietary supplementation of 25-hydroxy vitamin D3 starts a "vitamin D-cascade enrichment," allowing vitamin D to effect through genomic mechanism (via vitamin D receptor, a nuclear transcripton factor) and / or nongenomic signal transduction pathways to directly and indirectly regulate gene expression of myogenic regulatory proteins (MRFs) and Growth Factors (GFs) in enhancing muscle hyperplasia prenatally and muscle hypertrophy postnatally. 25-hydroxy vitamin D3, acting through MRFs and GFs may prenatally influence the determination step for commitment of the proliferating somatic cells to develop into the specific myogenic lineages of myoblast precursor cells (embryonic myoblasts, fetal myoblasts and in particular, the satellite cells) and to become "Vitamin D memory" myoblasts. The Vitamin D memory myoblasts are "switched on" by 25-hydroxyvitamin D3 to amplify their proliferation, differentiation and fusion with existing muscle fibers during postnatal muscle growth.

Thus one aspect of this invention is a method of promoting myogenesis in a mammal, comprising prenatally administering a sufficient amount of 25-hydroxy vitamin D3 to the mammal during a muscle fiber hyperplasia period. In preferred embodiments of this invention, the method of prenatally administering the 25-OH D3 is by oral administration to the pregnant mother of the mammal, such as buy incorporating 25-OH into the mother's feed. It has been found that this type of administration can affect muscle mass in the embryonic mammal. Another aspect of this invention is the use of 25-OH D3 to increase muscle fiber hyperplasia in an embryonic mammal.

The mammal of this invention can be any species. Preferred mammals according to this invention are humans, swine, bovines, equines, canines, felines, rabbits, caprines (goats) and ovines Preferably, the mammal is a pig.

For pregnant pigs, the dosage can range from about 10 µg to about 100 µg per kg pig feed diet, preferably from about 50 to about 80 µg 25-OH D3 per kg diet. For other mammals, the amounts are similar, and can be based on the amount of feed given per day. For optimum results, the 25-OH D3 supplementation should be part of a diet which contains an adequate supply of nutrients, generally known to be of benefit to the organism. For example in the pig, the B-group vitamins are essential and therefore they are routinely supplied to ensure all other nutrients like water, carbohydrates, proteins, fats, other vitamins, and minerals are efficiently utilized for production functions and maintenance.

The mammal preferably receives a prenatal dose of 25-Hydroxyvitamin D3 during the myogenesis period. It has also been found, in accordance with this invention, that if this animal which has been prenatally exposed to 25-OH D3 is then fed a diet which also contains a 25-Hydroxy Vitamin D3 supplementation, than its muscle mass is increased over similar animals which were fed a 25-OH D3 diet, but which did not receive the prenatal dosing of 25-hydroxy Vitamin D3. Thus, in particularly preferred embodiments of this invention, the mammal, preferably a pig receives both a prenatal supplement of 25-OH D3 (by feeding the mother) and is fed continuously during its life (after weaning) with a diet supplemented with 25-OH D3.

The following non-limiting Examples are presented to better define the invention.

### EXAMPLES

### EXAMPLE 1

### Swine Growth Performance

Study A. Two treatment groups were used in all studies. The control group contained first-to-fifth parity pigs (sows and progenies) fed rations without 25-OH D3 supplementation. The experimental group was made up of first-to-fifth parity pigs fed rations supplemented with 50 mcg 25-OH D3/kg diet) and farrowed to sows fed rations supplemented with 25-OH D3 since 6 months old. Study A only covered the pre-starter to finisher stage (41-150 days old) for the first to third parities pigs, while it covered the booster to finisher stage (7-150 days old) for the fourth and fifth parity pigs.

Study B. Two treatment groups were also used in this study. Pigs were randomly selected from mixed-parity sows fed rations containing no supplemental 25-OH on the same farm. The control group was fed rations without 25-OH D3, whereas the experimental group was fed rations supplemented with 25-OH D3 (50 mcg 25-OH D3/kg diet). Study B covered the pre-starter to finisher stage (41-150 days old).

Number of pigs and replicates: There were 8-11 replicates per treatment and 4 pigs per replicate.

Randomization and Allotment of pigs: Pigs were grouped according to size and sex and randomly allocated to each treatment. The number of male-replicates for the two treatments were equalized.

Rations: The rations involved in all studies were gestating and lactation rations for the sows. Booster, pre-starter, starter, grower and finisher rations were given to the progenies. Feeding: The experimental pigs were fed *ad libitum* with dry feeds. Booster pigs (sucklings) were fed starting when they were 7 days old. Feeds were placed in a linear trough designed for piglets. For a few days, only a small amount of feed was given to the pigs, but when they were eating, the required amount was always in the feeder. The amounts of feeds were properly weighed and recorded. Pre-starter pigs were fed for 30 days using linear feeders while they were confined in slatted and elevated nursery pens. The grower-finisher pigs were fed using concrete linear feeders for 80 days while they were confined in pens with cement flooring. The following rations were fed to pigs corresponding to their age.

| Type of ration | Age (days) |
|---|---|
| Booster | 7-40 |
| Pre-starter | 41-70 |
| Starter | 71-100 |
| Grower | 101-130 |
| Finisher | 131-150 |

Other management Practices: All experimental animals were subject to the standard operating procedure in the farm where these studies were conducted. Data Collection: The following data were collected:
1. Weights of piglets at 7 days old
2. Weight of piglets at 28 days old
3. Weight of pigs at 41, 70, 100, 130, and 150 days old
4. Feed consumption during the following periods:
   a) booster period (7-28 days old; 29-40 days old)
   b) pre-starter period (41-70 days old)
   c) starter period (71-100 days old)
   d) grower period (101-130 days old)
   e) finisher period (131-150 days old)

### RESULTS:

Across parities, pigs weaned from 25-OH D3 -fed sows were fed 50 µg 25-OH D3/kg diet and those weaned from control sows were fed the control diet (containing no 25-OH D3). Tables 1-5, below show the growth performance of prestarting-to-finishing pigs for parities 1 to 5. Consistently across the three parities, pigs fed 25-OH D3 gained weight faster (a delta average of 3.9 kg) an converted feed to gain (a delta average of 7 points) more efficient than those fed the control diet.

**TABLE 1**

| Effects of 25-OH D3 on growth performance of first parity 41-to-150-day-old (prestarting-to-finishing) pigs farrowed to sows fed 25-OH D3. | | | | | | | |
|---|---|---|---|---|---|---|---|
| Treatment | Replicate | Initial body wt, kg. | Final body wt., kg | Total wt gain, kg | Daily wt gain, kg | Total feed intake, kg | Feed: Gain |
| Control | 11 | 7.60 | 82.06 | 74.47 | 0.68 | 183.74 | 2.47 |
| 25-OH D3 | 11 | 8.24 | 87.78 | 79.55 | 0.72 | 184,60 | 2.32 |
| 25-OH D3 v. control (δ) | | 0.64 | 5.72 | 5.08 | 0.04 | 0.86 | -0.15 |
| *P* value | | 0.17 | 0.05 | 0.07 | 0.07 | 0.89 | 0.02 |

**TABLE 2**

| Effects of 25-OH D3 on Growth Performance of second parity 41-to-150-day-old (prestarting-to-finishing) pigs farrowed to sows fed 25-OH D3. | | | | | | | |
|---|---|---|---|---|---|---|---|
| Treatment | Replicate | Initial Body wt, kg. | Final body wt., kg | Total wt gain, kg | Daily wt gain, kg | Total feed intake, kg | Feed: Gain |
| Control | 8 | 8.81 | 88.44 | 79.63 | 0.72 | 184.49 | 2.32 |
| 25-OH D3 | 10 | 10.56 | 95.78 | 85.22 | 0.78 | 193.74 | 2.28 |
| 25-OH D3 v. control (δ) | | 1.67 | 7.34 | 5.59 | 0.06 | 9.25 | -0.04 |
| *P* value | | 0.02 | 0.01 | 0.03 | 0.02 | 0.07 | 0.62 |

**TABLE 3**

| Effects of 25-OH on growth performance of third parity 41-150-day-old (prestarting -to-finishing) pigs farrowed to sows fed 25-OH D3 | | | | | | | |
|---|---|---|---|---|---|---|---|
| Treatment | Replicate | Initial Body wt, kg. | Final body wt., kg | Total wt gain, kg | Daily weight gain, kg | Total feed intake, kg | Feed: Gain |
| Control | 9 | 8.13 | 86.51 | 78.39 | 0.71 | 178.83 | 2.29 |
| 25-OH D3 | 9 | 8.88 | 92.39 | 83.51 | 0.76 | 179.35 | 2.16 |
| 25-OH D3 v. control (δ) | | 0.75 | 5.88 | 5.12 | 0.05 | 0.52 | -0.13 |
| P value | | 0.04 | 0.11 | 0.13 | 0.14 | 0.86 | 0.11 |

**TABLE 4**

| Effects of 25-OH on growth performance of fourth parity 41-150-day-old (prestarting -to-finishing) pigs farrowed to sows fed 25-OH D3 | | | | | | | |
|---|---|---|---|---|---|---|---|
| Treatment | Replicate | Initial Body wt, kg. | Final body wt., kg | Total weight gain, kg | Daily wt gain, kg | Total feed intake, kg | Feed: Gain |
| Control | 10 | 8.28 | 88.36 | 80.09 | 0.73 | 168.79 | 2.12 |
| 25-OH D3 | 10 | 8.43 | 89.57 | 81.15 | 0.74 | 166.93 | 2.07 |
| 25-OH D3 v. control (δ) | | 0.15 | 1.21 | 1.06 | 0.01 | -1.86 | -0.05 |
| *P* value | | 0.71 | 0.76 | 0.76 | 0.76 | 0.34 | 0.43 |

**TABLE 5**

| Effects of 25-OH on growth performance of fifth parity 41-150-day old (prestarting -to-finishing pigs) farrowed to sows fed 25-OH D3 | | | | | | | |
|---|---|---|---|---|---|---|---|
| GROWTH PERFORMANCE | | | | | | | |
| Treatment | Replicate | Initial Body wt, kg. | Final body wt., kg | Total wt gain, kg | Daily wt gain, kg | Total feed intake, kg | Feed: Gain |
| Control | 8 | 8.73 | 89.43 | 80.70 | 0.73 | 179.59 | 2.22 |
| 25-OH D3 | 8 | 8.66 | 91.94 | 83.27 | 0.76 | 184.63 | 2.22 |
| 25-OH D3 v. control (δ) | | -0.07 | 2.51 | 2.57 | 0.03 | 5.04 | 0.00 |
| *P* value | | 0.85 | 0.24 | 0.19 | 0.17 | 0.24 | 0.38 |

### EXAMPLE 2

In this study, the pigs that were used were weaned from sows that were not fed 25-OH D3. These pigs were observed to gain weight at a similar rate to those fed without 25-OH D3, but the former converted feed to weight gain more efficiently than the latter, as seen in Table 6, below.

**Table 6**

| Effect of 25-OH D3 on growth performance of mixed-parity 41-to-150-day old prestarting-to-finishing pigs farrowed to sows fed without 25-OH D3 | | | | | | | |
|---|---|---|---|---|---|---|---|
| Treatment | Replicate | Initial body wt, kg. | Final body wt., kg | Total wt gain, kg | Daily wt gain, kg | Total feed intake, kg | Feed: Gain |
| Control | 10 | 8.33 | 88.61 | 80.27 | 0.730 | 190.94 | 2.40 |
| 25-OH D3 | 10 | 8.44 | 89.59 | 81.15 | 0.74 | 190.05 | 2.34 |
| 25-OH D3 v. control (δ) | | 0.11 | 0.98 | 0.88 | 0.01 | -0.89 | -0.06 |
| *P* value | | 0.84 | 0.67 | 0.69 | 0.69 | 0.67 | 0.52 |

Pigs weaned from 25-OH D3-fed sows, but which were not fed 25-OH D3 were observed to gain weight slower than those fed 25-OH D3 (data from farm records, n=23 pigs; body weight average = 80.90 kg); see Table 1. They also gained weight at a similar weight to control pigs (of Table 1) and those weaned from sows not fed 25-OH D3 (Table 6).

## Claims

1. Use of 25-hydroxy vitamin D3 for non-therapeutically promoting myogenesis in a mammal by prenatally administering a sufficient amount of 25-hydroxy vitamin D3 to the mammal during a muscle fiber hyperplasia period.

2. Use according to claim 1 wherein the prenatally administering comprises administering to the pregnant mother.

3. Use according to claim 2, wherein the prenatally administering is oral.

4. Use according to claim 1, wherein the prenatally administering is by incorporating 25-hydroxy vitamin D3 into the mother's feed.

5. Use according to claim 1, wherein the mammal is selected from the group consisting of: humans, swine, bovines, equines, canines, felines, caprines, rabbits, and ovines.

6. Use according to claim 5, wherein the mammal is a pig.

7. Use according to claim 5, wherein the amount of 25-hydroxy vitamin D3 administered to the pregnant sow is from about 10µg to about 100µg per kg feed.

8. Use according to claim 7, wherein the amount is from about 50 µg to about 80 µg per kg feed.

9. Use according to claim 7, wherein the sow is fed 25-hydroxy vitamin D3 starting at 6 months of age.

## Patentansprüche

1. Verwendung von 25-Hydroxy-Vitamin D3 für die nichttherapeutische Förderung der Myogenese in einem Säuger durch die pränatale Verabreichung einer ausreichenden Menge an 25-Hydroxy-Vitamin D3 an das Säuger während einer Muskelfaserhyperplasie-Periode.

2. Verwendung nach Anspruch 1, wobei die pränatale Verabreichung die Verabreichung an die schwangere Mutter umfasst.

3. Verwendung nach Anspruch 2, wobei die pränatale Verabreichung oral erfolgt.

4. Verwendung nach Anspruch 1, wobei die pränatale Verabreichung durch Einarbeiten von 25-Hydroxy-Vitamin D3 in die Nahrung der Mutter erfolgt.

5. Verwendung nach Anspruch 1, wobei das Säuger aus der Gruppe bestehend aus Menschen, Schweinen, Bovidae, Equidae, Canidae, Felidae, Capridae, Kaninchen und Ovidae ausgewählt ist.

6. Verwendung nach Anspruch 5, wobei es sich bei dem Säuger um ein Schwein handelt.

7. Verwendung nach Anspruch 5, wobei die der trächtigen Sau verabreichte Menge an 25-Hydroxy-Vitamin D3 ungefähr 10 µg bis ungefähr 100 µg pro kg Futter beträgt.

8. Verwendung nach Anspruch 7, wobei die Menge ungefähr 50 µg bis ungefähr 80 µg pro kg Futter beträgt.

9. Verwendung nach Anspruch 7, wobei der Sau 25-Hydroxy-Vitamin D3 ab dem Lebensalter von 6 Monaten verfüttert wird.

## Revendications

1. Utilisation de 25-hydroxy-vitamine D3 pour stimuler de façon non thérapeutique la myogenèse chez un mammifère par administration prénatale d'une quantité suffisante de 25-hydroxy-vitamine D3 au mammifère pendant une période d'hyperplasie des fibres musculaires.

2. Utilisation selon la revendication 1 dans laquelle l'administration prénatale comprend l'administration à la mère enceinte.

3. Utilisation selon la revendication 2, dans laquelle l'administration prénatale est orale.

4. Utilisation selon la revendication 1, dans laquelle l'administration prénatale est par incorporation de 25-hydroxy-vitamine D3 dans l'alimentation de la mère.

5. Utilisation selon la revendication 1, dans laquelle le mammifère est choisi dans le groupe constitué de : humains, porcs, bovins, équins, canins, félins, caprins, lapins, et ovins.

6. Utilisation selon la revendication 5, dans laquelle le mammifère est un porc.

7. Utilisation selon la revendication 5, dans laquelle la quantité de 25-hydroxy-vitamine D3 administrée à la truie gravide est d'environ 10 µg à environ 100 µg par kg d'aliment.

8. Utilisation selon la revendication 7, dans laquelle la quantité est d'environ 50 µg à environ 80 µg par kg d'aliment.

9. Utilisation selon la revendication 7, dans laquelle la truie est alimentée avec de la 25-hydroxy-vitamine D3 à partir de l'âge de 6 mois.
